(19) Europäisches Patentamt

European Patent Office

Office européen des brevets

(11) Veröffentlichungsnummer : **0 160 688**
**B1**

(12) **EUROPÄISCHE PATENTSCHRIFT**

(45) Veröffentlichungstag der Patentschrift :
01.02.89

(51) Int. Cl.⁴ : **A 61 B 17/36**

(21) Anmeldenummer : **84904091.0**

(22) Anmeldetag : **29.10.84**

(86) Internationale Anmeldenummer :
**PCT/DE 84/00225**

(87) Internationale Veröffentlichungsnummer :
**WO/85018 (09.05.85 Gazettee 85/11)**

(54) **GEPULSTER LASER FÜR MEDIZINISCHE ANWENDUNGEN.**

(30) Priorität : **29.10.83 DE 3339370**

(43) Veröffentlichungstag der Anmeldung :
**13.11.85 Patentblatt 85/46**

(45) Bekanntmachung des Hinweises auf die Patenter-
teilung : **01.02.89 Patentblatt 89/05**

(84) Benannte Vertragsstaaten :
**AT BE CH DE FR GB LI NL SE**

(56) Entgegenhaltungen :
**EP--A-- 0 075 860**
**EP--A-- 0 089 921**

(73) Patentinhaber : **MEDITEC REINHARDT THYZEL GMBH**
**Obere Bergstrasse 3**
**D-8501 Heroldsberg (DE)**

(72) Erfinder : **SCHRÖDER, Eckhard**
**Hans Sachsstr. 9**
**D-8501 Eckental (DE)**
Erfinder : **THYZEL, Reinhardt**
**Obere Bergstr. 3**
**D-8501 Heroldsberg (DE)**

(74) Vertreter : **Steinmann, Otto C. et al**
**Kanzlei Münich, Steinmann, Schiller Willibaldstrasse 36**
**D-8000 München 21 (DE)**

**Beschreibung**

Technisches Gebiet

Die Erfindung bezieht sich auf einen gepulsten Laser für medizinische Anwendungen, insbesondere für ophtalmologische Behandlungen.

Als gepulster Laser können beispielsweise Neodym-YAG-Laser mit Mode-locking oder Cueswitching benutzt werden, aber auch andere gepulste Laser, z. B. Argon-Laser.

Stand der Technik

Gepulste Laser werden beispielsweise zu Operationen am menschlichen Auge eingesetzt. Bei den bekannten Lasern findet keine Kontrolle der Energie des Laserimpulses vor dem Auslösen eines Operationsimpulses statt. Es ist ohne weiteres einzusehen, daß, wenn die Energie des Laserimpulses zu groß ist, schwere Schäden an dem zu behandelnden Auge hervorgerufen werden können.

Darstellung der Erfindung

Der Erfindung liegt die Aufgabe zugrunde, einen gepulsten Laser für medizinische Anwendungen und insbesondere für ophtalmologische Behandlungen zu schaffen, bei dem die Energie der Laserimpulse kontrolliert werden kann.

Diese Aufgabe wird erfindungsgemäß durch die im kennzeichnenden Teil des Patentanspruchs 1 angegebenen Merkmale gelöst.

Erfindungsgemäß wird von einem gepulsten Laser ausgegangen, dessen Laserstrahlimpuls eine definierte Polarisationsebene aufweist und bei dem die Energie eines einzelnen Impulses durch Verdrehen eines Polarisationsteilerwürfels steuerbar ist (siche EP-A2-0075860). Bei derartigen Lasern gibt es wie erfindungsgemäß erkannt worden ist, folgende Ursachen für eine falsche Energieabgabe :

1. Der Polarisationsteilerwürfel befindet sich beispielsweise aufgrund von mechanischen Fehlern etc. nicht in seiner Sollstellung, so daß er nicht den gewünschten Bruchteil der Laserenergie durchläßt.

2. Die vom Laser während eines Impulses abgegebene Energie entspricht nicht der Sollenergie.

Erfindungsgemäß ist nun erkannt worden, daß die Energie zweier aufeinanderfolgender Laserimpulse bei den im Oberbegriff des Patentanspruchs 1 vorausgesetzten Lasern nie mehr als um ± 20 % schwankt. Bei medizinischen Anwendungen und insbesondere bei Operationen am menschlichen Auge sind derartige Abweichungen der tatsächlichen Energie von der Sollenergie tolerabel.

Damit kann — unabhängig davon, welche der beiden vorgenannten Ursachen für eine falsche Energieabgabe vorliegt — eine zu stark von der Sollenergie abweichende Energie des Behandlungsimpulses dadurch festgestellt werden, daß nach jedem Verdrehen des Polarisationsteilerwürfels und vor dem anschließenden Behandlungsimpuls ein Probeimpuls ausgelöst wird. Damit durch den Probeimpuls keine Schäden beispielsweise bei einem Patienten oder bei der Bedienungsperson hervorgerufen werden, erfolgt die Abgabe des Probeimpulses intern, d. h. im Lasergerät, da während des Auslösens des Probeimpulses ein Verschluß den nach außen gehenden Strahlengang schließt.

Vorteilhafte Weiterbildungen der Erfindung sind in den Unteransprüchen angegeben.

Eine besonders vorteilhafte Anwendung findet die Erfindung bei Neodym-YAG-Lasern, die häufig für Operationen am menschlichen Auge eingesetzt werden. Bei derartigen Lasern kann — je nach Art der Steuerung (passives Modelocking oder aktives bzw. passives Cue-switching) — die Energie in dem vorstehend angegebenen Rahmen schwanken. Durch die Abgabe eines « Probeschusses » oder gegebenenfalls mehrerer Probeimpulse vor dem nachfolgenden Behandlungsimpuls kann das Intervall, in dem die Energie schwanken kann, zuverlässig eingegrenzt werden.

Das erfindungsgemäß bereitgestellte Energie-Meßsignal für den Laserimpuls kann natürlich auch zur Regelung der Laserenergie herangezogen werden.

Diese Energieregelung kann beispielsweise durch Regelung des Drehwinkels des Polarisationsteilerwürfels (Anspruch 3) oder der Spannung der Laser-Blitzlampe (Anspruch 4) erfolgen. Die Regelung kann beispielsweise mit einer elektronischen Steuereinheit erfolgen, deren Aufbau für den Durchschnittsfachmann keine Schwierigkeit ist, so daß auf eine detaillierte Beschreibung verzichtet werden kann.

In den Ansprüchen 5 bis 7 sind weitere vorteilhafte Weiterbildungen der Erfindung angegeben, durch die die Betriebssicherheit des Lasers erhöht wird.

Die in Anspruch 5 gekennzeichnete selbsttätige « Nullstellung » des Polarisationsteilerwürfels nach dem Einschalten des Lasers zwingt die Bedienungsperson, die für einen Behandlungsvorgang erforderliche Laserenergie immer neu einzustellen. Hierdurch wird einerseits vermieden, daß eine zu hohe, noch von einem vorigen Behandlungsvorgang eingestellte Energie, ohne weiteres Nachdenken übernommen wird. Andererseits läßt sich die Steuerung des Geräts vereinfachen, da immer vor einem Behandlungsimpuls ein Verdrehen des Polarisationsteilerwürfels erfolgt, so daß ein Probeimpuls nur durch ein Verdrehen des Polarisationsteilerwürfels auslösbar sein muß.

Dabei ist es besonders vorteilhaft, wenn gemäß Anspruch 6 der Probeimpuls immer dann ausgelöst wird, wenn der zum mechanischen Verdrehen des Polarisationsteilerwürfels vorgesehene Bedienungsknopf losgelassen wird. Insbesondere bei Augenbehandlungen soll nämlich die Zeitverzögerung zwischen Auslösen des Behandlungsimpulses und Abgabe des Behandlungsimpulses sehr kurz sein, da die Bedienungsperson beispielsweise mittels einer Spaltlampe die Stellung des Auges überprüft, und in dem Moment, in dem das Auge die richtige Stellung einnimmt, den Behandlungsimpuls auslöst. Dabei wäre es von Nachteil, wenn vor dem Behandlungsimpuls erst noch ein « Probeschuß" ausgelöst werden müßte.

Der gemäß Anspruch 7 immer im Strahlengang verbleibende Strahlteiler verhindert etwaige Beeinflussungen der Laserenergie durch das Aus- bzw. Einschwenken des Strahlteilers.

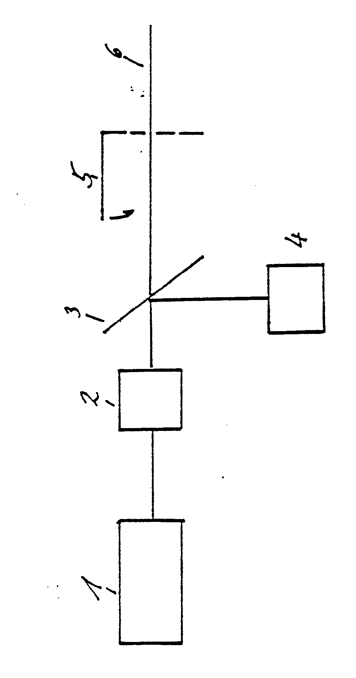

## Kurze Beschreibung der Zeichnung

Die Erfindung wird nachstehend anhand eines Ausführungsbeispiels unter Bezugnahme auf die Zeichnung näher beschrieben, deren einzige Figur den Grundaufbau eines erfindungsgemäß ausgebildeten gepulsten Lasers zeigt.

## Weg zur Ausführung der Erfindung

Der erfindungsgemäße Laser weist eine Laserröhre 1, einen Polarisationsteilerwürfel 2, einen Strahlteiler 3 und ein lichtempfindliches Element 4 sowie einen Verschluß 5 auf.

Die Laserröhre 1 kann beispielsweise ein Neodym-YAG-Laser sein, in den bereits ein Polarisator integriert ist, so daß die Laserröhre 1 einen polarisierten Laserstrahlimpuls mit einer definierten Polarisationsebene abgibt. Auf diese Weise kann durch mechanisches Verdrehen des Polarisationsteilerwürfels 2 die durch den Polarisationsteilerwürfel hindurchgehende Energie verstellt werden.

Die mechanische Drehung des Polarisationsteilerwürfels kann beispielsweise mit einem nichtdargestellten Betätigungsknopf erfolgen. Beim Loslassen des Betätigungsknopfes wird automatisch ein Probeimpuls ausgelöst, bei dem der Verschluß 5 den Behandlungsstrahlengang 6 schließt.

Liegt die gemessene Energie innerhalb einer bestimmten Toleranzschwelle, bei einem Neodym-YAG-Laser für medizinische Anwendungen beispielsweise innerhalb von $\pm\,20\,\%$ um den vorgegebenen Sollwert, so kann die Bedienungsperson einen Behandlungsimpuls auslösen. Andernfalls wird das Auslösen eines Behandlungsimpulses verhindert. Daneben ist es auch möglich, das Meßergebnis zur Regelung der Laserenergie, beispielsweise durch Regeln der Blitzlampenspannung zu verwenden.

Bei dem gezeigten .Ausführungsbeispiel verbleibt der Strahlteiler 3, der einen Teil des Laserstrahls auf das lichtempfindliche Element 4 umlenkt, auch dann im Behandlungsstrahlengang, wenn kein Probeimpuls ausgelöst wird. Da auf diese Weise der Behandlungsstrahlengang beim Auslösen eines Behandlungsimpulses gegenüber dem Strahlengang beim Auslösen eines Probeimpulses nicht verändert wird, wird die Betriebssicherheit weiter erhöht. Daneben ist es auch möglich, das lichtempfindliche Element beispielsweise auf dem Verschluß 5 vorzusehen, so daß ein Strahlteiler, der einen Teil des Laserstrahls auslenkt, nicht benötigt wird.

Die Steuerung kann eine beliebig aufgebaute elektronische Schaltung sein. Beispielsweise kann die Steuerung einen Mikrocomputer aufweisen, der beim Loslassen des Betätigungsknopfes für den Polarisationsteilerwürfel einen Probeimpuls auslöst, die Energie des Impulses in der vorstehend angegebenen Art mißt, und gegebenenfalls die Energie der Laserröhre entsprechend regelt.

## Patentansprüche

1. Gepulster Laser für medizinische Anwendungen, insbesondere für ophtalmologische Behandlungen, bei dem die Energie des Laserstrahlimpulses mit einer definierten Polarisationsebene durch Verdrehen eines Polarisators steuerbar ist, dadurch gekennzeichnet, daß eine Steuerung nach jedem Verdrehen des als Polarisationsteilerwürfels ausgebildeten Polarisators, in jedem Fall aber mindestens einmal vor jedem Behandlungsimpuls einen Probeimpuls auslöst, dessen Energie ein lichtempfindliches Element (4) mißt, und daß ein Verschluß (5) den noch außen gehenden Behandlungsstrahlengang gang (6) während des Probeimpulses schließt.

2. Laser nach Anspruch 1, dadurch gekennzeichnet, daß der Laser (1) ein Neodym-YAG-Laser ist.

3. Laser nach Anspruch 1, dadurch gekennzeichnet, daß das Ausgangssignal des lichtempfindlichen Elements (4) zur Regelung der Energie der Laserimpulse und/oder zur selbsttätigen Einstellung des Drehwinkels des Polarisationsteilerwürfels dient.

4. Laser nach Anspruch 3, dadurch gekennzeichnet, daß die Spannung der Laser-Blitzlampe entsprechend dem Ausgangssignal des lichtempfindlichen Elements (4) regelbar ist.

5. Laser nach Anspruch 1, dadurch gekennzeichnet, daß nach dem Anschalten des Lasers die Steuerung den Polarisationsteilerwürfel (2) in die Stellung dreht, in der er die minimale Laserenergie durchläßt.

6. Laser nach Anspruch 5, dadurch gekennzeichnet, daß der Polarisationsteilerwürfel von einer Bedienungsperson mittels eines Betätigungsknopfes verdrehbar ist, und daß die Steuerung den Probeimpuls selbsttätig auslöst, wenn der Betätigungsknopf losgelassen wird.

7. Laser nach Anspruch 1, dadurch gekennzeichnet, daß ein fest im Strahlengang (6) des Laserstrahls angeordneter Strahlteiler (3) einen Teil des Laserstrahls auf das lichtempfindliche Element (4) auslenkt.

**Claims**

1. Pulsed laser for medical applications, in particular ophthalmologic treatments, wherein the energy of the laser beam pulse with a defined plane of polarization is controllable by the rotation of a polarizer, characterized in that each time after said polarizer, which is designed as a polarizing splitter cube, has been rotated, but in any case at least once prior to each treatment pulse, a control system triggers a test pulse whose energy is measured by a photosensitive element (4), and that a shutter (5) closes the optical path of the outwardly directed treatment beam (6) during said test pulse.

2. Laser in accordance with Claim 1, characterized in that said laser (1) is of the neodymium-doped YAG type.

3. Laser in accordance with Claim 1, characterized in that the output signal of said photosensitive element (4) serves to control the energy of the laser pulses and/or for automatic setting of the angle of rotation of said polarizing splitter cube.

4. Laser in accordance with Claim 3, characterized in that the voltage of the laser flash lamp can be controlled in accordance with the output signal of said photosensitive element (4).

5. Laser in accordance with Claim 1, characterized in that following laser switch-on, said control system rotates said polarizing splitter cube (2) into the position in which it allows the minimum laser energy to pass.

6. Laser in accordance with Claim 1, characterized in that said polarizing splitter cube can be rotated by an operator by means of a control button, and that said control system automatically triggers said test pulse as soon as said control button is released.

7. Laser in accordance with Claim 1, characterized in that a beam splitter (3) fixedly arranged in the optical path (6) of the laser beam deflects part of said laser beam towards said photosensitive element (4).

**Revendications**

1. Laser en régime pulsé pour applications médicales, en particulier pour des traitements ophthalmologiques, dans lequel l'énergie de l'impulsion du faisceau laser peut être commandée par un plan de polarisation en tournant un polariseur, caractérisé en ce que chaque fois que le polariseur en forme d'un cube de fractionnement de polarisation a été tourné, mais en tout cas une fois avant chaque impulsion de traitement, un système de commande déclenche une impulsion d'essai dont l'énergie est mesurée par un élément photosensible (4), et en ce qu'un obturateur (5) ferme la marche du faisceau de traitement (6) passant à l'extérieur, pendant la durée de l'impulsion d'essai.

2. Laser selon la revendication 1, caractérisé en ce que le laser (1) est du type YAG dopé au Néodyme.

3. Laser selon la revendication 1, caractérisé en ce que le signal de sortie dudit élément photosensible (4) sert à l'asservissement de l'énergie des impulsions de laser et/ou au réglage automatique de l'angle d'orientation dudit cube de fractionnement de polarisation.

4. Laser selon la revendication 3, caractérisé en ce que la tension de la lampe par éclair du laser est pourvue pour être asservie en correspondance avec le signal de sortie dudit élément photosensible (4).

5. Laser selon la revendication 1, caractérisé en ce qu'après la mise en circuit du laser, le système de réglage tourne ledit cube de fractionnement de polarisation (2) dans une position dans laquelle il laisse passer l'énergie minimale du laser.

6. Laser selon la revendication 5, caractérisé en ce qu'un bouton de commande est pourvu pour un opérateur tournant ledit cube de fractionnement de polarisation, et en ce que le système de commande déclenche l'impulsion d'essai automatiquement dès que ledit bouton de commande est relâché.

7. Laser selon la revendication 1, caractérisé en ce qu'une lame séparatrice (3) fixe est montée dans la marche (6) du faisceau laser pour la déviation d'une part du faisceau laser vers ledit élément photosensible (4).